# EUROPEAN PATENT SPECIFICATION

(11) **EP 2 515 856 B1**
(45) Date of publication and mention of the grant of the patent: **02.04.2014**
(21) Application number: 10800932.5
(22) Date of filing: 22.12.2010
(51) Int. Cl.: A61K 9/12, A61P 11/06, A61P 11/00, A61K 45/06, A61K 9/00, A61K 31/40

(54) **Aerosol Formulation for COPD**
Aerosol-Formulierung für COPD
Formule d'aérosol pour COPD

(30) Priority: 23.12.2009 EP 09180662
(43) Date of publication of application: 31.10.2012
(73) Proprietor: Chiesi Farmaceutici S.p.A., 43100 Parma (IT)
(72) Inventor: BONELLI, Sauro, I-43100 Parma (IT); USBERTI, Francesca, I-43100 Parma (IT); ZAMBELLI, Enrico, I-43100 Parma (IT)
(74) Representative: Minoja, Fabrizio
(86) International application number: PCT/EP2010/070476
(87) International publication number: WO 2011/076840

(56) References cited:
- WO-A1-2008/000482
- WO-A2-2005/074900
- US-A1- 2004 101 483
- US-A1- 2006 257 324
- "Glycopyrronium Bromide" In: "Martindale The complete drug reference", 1 January 2002 (2002-01-01), Pharmaceutical Press, XP002579323, ISBN: 0853694990 vol. 33, pages 467-467, the whole document

## Description

### FIELD OF THE INVENTION

The present invention relates to pharmaceutical aerosol solution formulations comprising glycopyrronium chloride, intended for use in pressurized metered dose inhalers. The invention further relates to use of such formulations in the prevention and therapy of respiratory disorders, including COPD.

### BACKGROUND OF THE INVENTION

Glycopyrronium bromide (also known as glycopyrrolate) is a muscarinic M3 anticholinergic agent used to reduce salivation associated with administration of certain anaesthetics, and as adjunctive therapy for peptic ulcers. It has also been reported to be effective in the treatment of asthmatic symptoms (Hansel et al., Chest 2005; 128:1974-1979).

WO 2005/107873 relates to the use of glycopyrrolate for the treatment of childhood asthma.

WO 01/76575 discloses a controlled release formulation for pulmonary delivery of glycopyrrolate. The formulation is intended for use in treatment of respiratory disease, in particular chronic obstructive pulmonary disease (COPD). The application focuses on dry powder formulations suitable for delivery by means of a dry powder inhaler (DPI).

Other counterions (including *inter alia* the chloride ion) have been mentioned as possible alternatives to the bromide counterion of glycopyrronium. WO 2006/100453 proposes the use of the iodide, acetate and sulphate salts as an alternative to glycopyrronium bromide due to milling difficulties associated with the latter.

Until the present disclosure there was no published evidence that glycopyrronium chloride is either clinically effective or capable of being formulated in a manner suitable for administration to patients with respiratory disease.

US 2004/101483 A1 in Example 4 discloses the preparation of an aerosol formulation (suspension) wherein to micronized glycopyrronium bromide and a solid auxiliary agent such as magnesium stearate, a HFA 227 plus HFA 134a mixture, treated beforehand with 1.4% w/w ethanol, is added.

Martindale Jan. 2002 monograph on glycopyrronium bromide shows that in investigations on compatibility of this substance with infusion solutions for injections and additives showed that the stability of glycopyrronium bromide is questionable above a pH of 6 owing to ester hydrolysis.

WO 2008/000482 A1 discloses a process for preparing dry powder formulations of a glycopyrronium salt with an antiadherent agent (metal stearate -and/or crystalline sugar) and admixing carrier particles.

WO 2005/074900 A2 discloses an inhalable combination of an anticholinergic and a beta-2 minetic for the treatment of inflammatory or obstructive respiratory diseases, such as COPD. In the description racemic glycopyrrolate, one of its enantiomers (R,R-glycopyrrolate) or diastereoisomers and salts are cited in general, however, in the examples only the DPI formulation and the pMDI suspension formulation of the R,R-enantiomer in combination with formoterol are disclosed.

US 2006/0257324 discloses the delivery of a combination of two or more dissolved drugs in a HFA propellant-cosolvent system with substantially the same particle size distribution allowing their co-deposition in the same lung region tract. The formulation comprises a beta-2agonist (formoterol or carmoterol exemplified) and a corticosteroid (beclometasone dipropionate exemplified), or an anticholinegic such as ipratropium, oxitropium, tiotropium or glycopyrronium bromide (only generically cited).

The present inventors have observed that glycopyrronium chloride has several advantages over glycopyrronium bromide with respect to pharmaceutical formulations. In particular, glycopyrronium chloride has better solubility properties than glycopyrronium bromide, and it has also been found to have better compatibility with other active ingredients, especially with formoterol.

It would be desirable to provide a clinically useful aerosol product in the form of a solution that delivers the now proven therapeutic benefits of glycopyrronium chloride in effective and consistent doses over an extended product lifetime, and ideally without the need for storage under special conditions of temperature or humidity.

### SUMMARY OF THE INVENTION

The present invention provides a pharmaceutical composition comprising glycopyrronium chloride at a dosage in the range of 0.5-100 µg per actuation dissolved in an HFA propellant and a co-solvent, characterized in that said composition contains an amount of 1 M hydrochloric acid (HCl) in the range of 0.05-0.4 µg/µl. Additional pharmaceutically active ingredients may also be included

In a further aspect the invention provides a pressurized metered dose inhaler or other container suitable for aerosol delivery, comprising the pharmaceutical composition of the invention.

In another aspect the invention provides the use of pharmaceutical compositions as described herein for the therapeutic or palliative treatment or prevention of respiratory disease conditions, such as COPD.

### DETAILED DESCRIPTION OF THE PREFERRED EMBODIMENTS

A solution formulation of glycopyrronium chloride in HFA propellant with ethanol as co-solvent was prepared and checked for stability after 3 months following storage under different conditions of temperature and humidity. One batch was stored under optimal conditions (refrigeration); the other batches were stored under accelerated degradation conditions of high temperature and humidity. Although the refrigerated batch remained stable over the 3 month period, the other batches degraded significantly over that time-span.

This is the first time that it has been attempted to formulate glycopyrronium chloride in an aerosol solution. This simple aerosol solution formulation of glycopyrronium chloride dissolved in propellant and co-solvent fails to meet the requirements for practical use, namely that it should be capable of being carried on the person without refrigeration and yet deliver consistent dosages of active ingredient.

The present inventors were able to overcome these stability issues by inclusion of a specific amount of inorganic acid in the formulation. In particular, they found that inclusion of an amount of 1M hydrochloric acid (HCl) in the range of 0.05-0.4 µg/µl, preferably 0.1-0.3 µg/µl, more preferably 0.19-0.25 µg/µl, optionally 0.21-0.23 µg/µl of the solution is sufficient to eliminate degradation of glycopyrronium chloride over an extended period of non-optimal storage, thereby ensuring a consistent dose of glycopyrronium chloride per actuation of the pMDI containing the solution formulation.

Glycopyrronium chloride, chemically defined as 3-[(cyclopentylhydroxyphenylacetyl)oxy]-1,1,-dimethylpyrrolidinium chloride, has two chiral centres corresponding to four potential different stereoisomers with configuration (3R,2'R)-, (3S,2'R)-, (3R,2'S)- and (3S,2'S)-. Glycopyrronium chloride in the form of any of these pure enantiomers or diastereomers or any combination thereof may be used in practicing the present invention. In one embodiment of the invention the (3S,2'R),(3R,2'S)-3-[(cyclopentylhydroxyphenylacetyl)oxy]-1,1-dimethylpyrrolidinium chloride racemic mixture is preferred. Glycopyrronium chloride is present in the formulation in an amount in the range from 0.005 to 0.83% (w/w), preferably from 0.010 to 0.13% (w/w), more preferably from 0.015 to 0.04% (w/w), wherein% (w/w) means the amount by weight of the component, expressed as percent with respect to the total weight of the composition.

Glycopyrronium chloride can be prepared using any suitable synthesis technique, such as that described in a co-pending application filed by Chiesi Farmaceutici SpA.

The propellant component of the composition may be any pressure-liquefied propellant but is preferably a hydrofluoroalkane (HFA) or a mixture of different HFAs, more preferably selected from the group consisting of HFA134a (1,1,1,2-tetrafluoroethane), HFA 227 (1,1,1,2,3,3,3-heptafluoropropane), and mixtures thereof. The preferred HFA is HFA134a. HFAs may be present in the formulation in an amount in the range from 75 to 95% (w/w), preferably from 85 to 90% (w/w).

The co-solvent incorporated into formulations of the invention has a higher polarity than that of the propellant and may include one or more substances such as a pharmaceutically acceptable alcohol, in particular ethanol, or a polyol such as propylene glycol or polyethylene glycol. Advantageously the co-solvent is selected from the group of lower branched or linear alkyl (C₁-C₄) alcohols such as ethanol and isopropyl alcohol. Preferably the co-solvent is ethanol.

The concentration of the co-solvent will vary depending on the final concentration of the active ingredient in the formulation and on the type of propellant. For example ethanol may be used in a concentration comprised in the range from 5 to 25% (w/w), preferably from 8 to 20% (w/w), more preferably from 10 to 15% (w/w). In one of the preferred embodiments the concentration of ethanol is 12% (w/w).

The ratio of propellant to co-solvent in the formulation is in the range 50:50 to 95:5 (w/w).

The pharmaceutically active components of the composition are preferable completely and homogeneously dissolved in the mixture of propellant and co-solvent, i.e. the composition is preferably a solution formulation.

Optionally the solution formulation compositions may comprise other known pharmaceutical excipients or additives, such as one or more low-volatility components in order to either increase the mass median aerodynamic diameter (MMAD) of the aerosol particles upon actuation of the inhaler and/or to improve the solubility of the active ingredient in the propellant/co-solvent mixture.

The low volatility component, when present, has a vapour pressure at 25°C lower than 0.1 kPa, preferably lower than 0.05 kPa.

Examples of low-volatility components may be esters such as isopropyl myristate, ascorbyl myristate, tocopherol esters; glycols such as propylene glycol, polyethylene glycol, glycerol; or surface active agents such as a saturated organic carboxylic acid (i.e. lauric, myristic, stearic acid) or an unsaturated carboxylic acid (i.e. oleic or ascorbic acid).

The amount of low volatility component may vary from 0.1 to 10% w/w, preferably from 0.5 to 5% (w/w), more preferably between 1 and 2% (w/w).

In one embodiment of the invention an amount of water comprised between 0.005 and 0.5% (w/w), and preferably up to 0.2% (w/w) may optionally be added to the formulations in order to favourably affect the solubility of the active ingredient without increasing the MMAD of the aerosol droplets upon actuation.

Advantageously, the formulations of the invention are free of other excipients such as surfactants besides the co-solvent, the propellant and a stabilizing amount of an acid.

The invention also relates to a method for preparing a pharmaceutical composition, comprising adding 1M HCl to a solution of glycopyrronium chloride in HFA propellant and co-solvent, wherein the amount of 1M HCl added is in the range of 0.05-0.4 µg per µl of the final solution.

The pharmaceutical compositions of the invention may further comprise other, additional pharmaceutically active agents for separate, sequential or simultaneous use. Optional additional pharmaceutically active components of the composition include any known compound for the prophylaxis or treatment of respiratory diseases and their symptoms. Examples of these active components are: beta-2-agonists such as formoterol, salbutamol, fenoterol, carmoterol (TA2005), indacaterol, milveterol, vilanterol (GSK 642444), terbutaline, salmeterol, bitolterol, and metaproterenol all in form of single stereoisomers or mixtures thereof and salts thereof; corticosteroids such as beclometasone dipropionate, fluticasone propionate, butixocort, mometasone furoate, triamcinolone acetonide, budesonide and its 22R-epimer, ciclesonide, flunisolide, loteprednol, and rofleponide; other anti-muscarinic drugs such as methscopolamine, ipratropium bromide, oxitropium bromide and tiotropium bromide; phosphodiesterase IV inhibitors such as: cilomilast, roflumilast, and tetomilast. Among these additional active components formoterol fumarate is particularly preferred.

The compositions of the invention can be inhaled from any suitable known MDI device. Desired doses of the individual pharmaceutically active components of the formulation are dependent on the identity of the component and the type and severity of the disease condition, but are preferably such that a therapeutic amount of the active ingredient is delivered in one or two actuations. Generally speaking, doses of active ingredient are in the range of about 0.5 µg-1mg per actuation, e.g. about 1-100 µg/actuation, and sometimes about 5-50 µg/actuation. The skilled person in the field is familiar with how to determine the appropriate dosage for each individual pharmaceutically active ingredient.

With specific reference to glycopyrronium chloride, the preferred dosage is about 0.5-100 µg per actuation, preferably about 1-40 µg per actuation, and more preferably about 5-26 µg per actuation, even more preferably 25 µg per actuation.

The pharmaceutical formulation of the invention is filled into known pMDI devices. Said devices comprise a canister fitted with a metering valve. Actuation of the metering valve allows a small portion of the spray product to be released.

Part or all of the canister may be made of a metal, for example aluminium, aluminium alloy, stainless steel or anodized aluminium. Alternatively the canister may be a plastic cans or a plastic-coated glass bottle.

The metal canisters may have part or all of the internal surfaces lined with an inert organic coating. Examples of preferred coatings are epoxy-phenol resins, perfluorinated polymers such as perfluoroalkoxyalkanes, perfluoroalkoxyalkylenes, perfluoroalkylenes such as poly-tetrafluoroethylene (Teflon), fluorinated-ethylene-propylene (FEP), polyether sulfone (PES) or fluorinated-ethylene-propylene polyether sulfone (FEP-PES) mixtures or combination thereof. Other suitable coatings could be polyamide, polyimide, polyamideimide, polyphenylene sulfide or their combinations. In certain embodiments canisters having the internal surface lined with FEP-PES or Teflon may preferably be used.

In other particular embodiments canisters made of stainless steel may be used.

The container is closed with a metering valve for delivering a daily therapeutically effective dose of the active ingredient. Generally the metering valve assembly comprises a ferrule having an aperture formed therein, a body moulding attached to the ferrule which houses the metering chamber, a stem consisting of a core and a core extension, an inner- and an outer- seal around the metering chamber, a spring around the core, and a gasket to prevent leakage of propellant through the valve.

The gasket seal and the seals around the metering valve may comprise elastomeric material such as EPDM, chlorobutyl rubber, bromobutyl rubber, butyl rubber, or neoprene. EPDM rubbers are particularly preferred. The metering chamber, core and core extension are manufactured using suitable materials such as stainless steel, polyesters (e.g. polybutyleneterephthalate (PBT)), or acetals. The spring is manufactured in stainless steel eventually including titanium. The ferrule may be made of a metal, for example aluminum, aluminum alloy, stainless steel or anodized aluminum. Suitable valves are available from manufacturers such as Valois, Bespak plc and 3M-Neotechnic Ltd.

The pMDI is actuated by a metering valve capable of delivering a volume of between 25-100 µl, preferably between 40-70 µl, and optionally about 50 µl, or about 63 µl per actuation.

Each filled canister is conveniently fitted into a suitable channeling device prior to use to form a metered dose inhaler for administration of the medicament into the lungs of a patient. Suitable channeling devices comprise, for example, a valve actuator and a cylindrical or cone-like passage through which medicament may be delivered from the filled canister via the metering valve to the mouth of a patient e.g. a mouthpiece actuator.

In a typical arrangement the valve stem is seated in a nozzle block which has an orifice leading to an expansion chamber. The expansion chamber has an exit orifice which extends into the mouthpiece. Actuator (exit) orifices having a diameter in the range 0.15 - 0.45 mm and a length from 0.30 to 1.7 mm are generally suitable. Preferably an orifice having a diameter from 0.2 to 0.44 mm is used, e.g. 0.22 0.25, 0.30, 0.33 or 0.42 mm.

In certain embodiments of the invention, it may be useful to utilize actuator orifices having a diameter ranging from 0.10 to 0.22 mm, in particular from 0.12 to 0.18 mm, such as those described in WO 03/053501. The use of said fine orifices may also increase the duration of the cloud generation and hence, may facilitate the coordination of the cloud generation with the slow inspiration of the patient.

In case the ingress of water into the formulation is to be avoided, it may be desired to overwrap the MDI product in a flexible package capable of resisting water ingress. It may also be desirable to incorporate a material within the packaging which is able to adsorb any propellant and co-solvent which may leak from the canister (e.g. a molecular sieve).

Optionally the MDI device filled with the formulation of the invention may be utilized together with suitable auxiliary devices favoring the correct use of the inhaler. Said auxiliary devices are commercially available and, depending on their shape and size, are known as "spacers", "reservoirs" or "expansion chambers". Volumatic^{™} is, for instance, one of the most widely known and used reservoirs, while Aerochamber^{™} is one of the most widely used and known spacers. A suitable expansion chamber is reported, for example, in WO 01/49350.

The formulation of the invention may also be used with common pressurized breath-activated inhalers such as those known with the registered names of Easi-Breathe^{™} and Autohaler^{™}.

The efficacy of an MDI device is a function of the dose deposited at the appropriate site in the lungs. Deposition is affected by the aerodynamic particle size distribution of the formulation which may be characterised *in vitro* through several parameters.

The aerodynamic particle size distribution of the formulation of the invention may be characterized using a Cascade Impactor according to the procedure described in the European Pharmacopoeia 6th edition, 2009 (6.5), part 2.09.18. An Apparatus E, operating at a flow rate range of 30 litres/min to 100 litres/min or an Apparatus D -Andersen Cascade Impactor (ACI)-, operating at a flow rate of 28.3 1/min, may be utilized. Deposition of the drug on each ACI plate is determined by high performance liquid chromatography (HPLC).

The following parameters of the particles emitted by a pressurized MDI may be determined:
i) mass median aerodynamic diameter (MMAD) is the diameter around which the mass aerodynamic diameters of the emitted particles are distributed equally;
ii) delivered dose is calculated from the cumulative deposition in the ACI, divided by the number of actuations per experiment;
iii) respirable dose (fine particle dose = FPD) is obtained from the deposition from Stages 3 (S3) to filter (AF) of the ACI, corresponding to particles of diameter ≤ 4.7 microns, divided by the number of actuations per experiment;
iv) respirable fraction (fine particle fraction=FPF) which is the percent ratio between the respirable dose and the delivered dose.
v) "superfine" dose is obtained from the deposition from Stages 6 (S6) to filter, corresponding to particles of diameter ≤ 1.1 microns, divided by the number of actuations per experiment.

The solutions of the invention are capable of providing, upon actuation of the pMDI device in which they are contained, a total FPF higher than 40%, preferably higher than 50%, more preferably higher than 60%.

Moreover the formulations of the invention are capable of providing, on actuation, a fraction higher than or equal to 30% of emitted particles of diameter equal to or less than 1.1 microns as defined by the content stages S6-AF of an Andersen Cascade Impactor, relative to the total fine particle dose collected in the stages S3-AF of the impactor. Preferably the fraction of emitted particles of diameter equal to or less than 1.1 microns is higher than or equal to 40%, more preferably higher than 50%, even more preferably higher than 60%, most preferably higher than 70%.

According to a further aspect of the invention there is provided a method of filling an aerosol inhaler with a composition of the invention. Known conventional bulk manufacturing methods and machinery in the field of pharmaceutical aerosol manufacture may be employed for the preparation of large scale batches for the commercial production of filled canisters.

The method comprises:
a) preparing a solution comprising glycopyrronium chloride, a co-solvent (e.g. ethanol), a mineral acid, a propellant comprising a HFA and optionally a low volatility component at a temperature from -50 to -60°C at which the solution does not vaporize;
b) cold filling the inhaler with the prepared solution; and
c) placing the valve onto the can and crimping.

An alternative method comprises:
a) preparing a solution comprising glycopyrronium chloride, a co-solvent (e.g. ethanol), a mineral acid, and optionally a low volatility component;
b) filling the open can with the bulk solution;
c) placing the valve onto the can and (vacuum) crimping; and
d) pressure-filling the can with HFA propellant through the valve

A further alternative method comprises:
a) preparing a solution comprising glycopyrronium chloride, a co-solvent (e.g. ethanol), a mineral acid, an optional low volatility component and HFA propellant using a pressurised vessel:
b) placing the valve onto the empty can and crimping; and
c) pressure-filling the can with the final solution formulation through the valve.

The packaged formulations of the invention are stable for extended periods of time when stored under normal conditions of temperature and humidity. In a preferred embodiment the packaged formulations are stable for at least 6 months at 25°C and 60% RH, more preferably for at least 1 year, most preferably for at least 2 years. Stability is assessed by measuring content of residual active ingredient. A "stable" formulation as defined herein means one retaining at least about 85%, preferably at least about 90%, and most preferably at least about 95% of residual content of each active ingredient at a given time point, as measured by HPLC-UV VIS.

The optimized stable formulations meet the specifications required by the ICH Guideline Q1B relevant for drug product stability testing for the purposes of drug registration.

The product of the invention may be used for prophylactic purposes or for symptomatic relief for a wide range of respiratory disorders, such as asthma of all types and chronic obstructive pulmonary disease (COPD).

Other respiratory disorders for which the pharmaceutical compositions of the invention may be beneficial are those characterized by obstruction of the peripheral airways as a result of inflammation and presence of mucus, such as chronic obstructive bronchiolitis, chronic bronchitis, emphysema, acute lung injury (ALI), cystic fibrosis, rhinitis, and adult or acute respiratory distress syndrome (ARDS).

### EXAMPLE

### Glycopyrronium chloride stability during storage

Solution formulations are prepared with the compositions shown in the following Table 1.

**Table 1**

| | Theoretical Unit Formula (µg/actuation for a 63 µl valve) | | | | |
|---|---|---|---|---|---|
| | **Glycopyrronium chloride (GLY)** | **Anhydrous ethanol** | **1M HCl** | **HFA 134a** | **Total** |
| Without Acid | 25 | 8856 | - | 64919 | 73800 |
| With Acid | 25 | 8856 | 14 | 64905 | 73800 |

The samples containing acid are formulated by the addition of 1M HCl in an amount corresponding to 0.222 µg/µl of the solution.

The solution is filled into canisters which are stored inverted under different conditions: 5°; 25°C/60% RH; 30°C/75% RH. The samples are analyzed chromatographically for glycopyrronium chloride content after 1, 2, and 3 months of storage.

The results show the stabilizing effect of the acid addition upon the glycopyrronium chloride solution formulations.

The formulation is found to maintain a constant content in the presence of 1M HCl, but to be highly dependent on time and temperature of storage if the acid is omitted. See in the following Table 2 the data when the formulation was stored for 3 months at 25°C/60% relative humidity with or without the acid.

**Table 2**

| Active ingredient | Residual % amount + standard deviation | Number of cans (N.) |
|---|---|---|
| Gly (without acid) | 90.3 + 1.1 | 2 |
| Gly (with acid) | 95.5 ± 1.3 | 3 |

The formulation containing GLY is found to maintain a constant content in the presence of 1M HCl, but to be highly dependent on time and temperature of storage if the acid is omitted. See in the following Table 3 the data for the total percent amount of impurities and/or degradation products expressed versus the initial amount of active ingredient when the single agent formulation was stored for 3 months at 40°C/75% relative humidity with or without the same amount of acid. The formulations were tested by a standard HPLC/UV VIS method for non-chiral impurities and degradation products of the active ingredient.

**Table 3**

| Active ingredient | Total impurities % Vs active ingredient | Number of cans (N.) |
|---|---|---|
| Gly (without acid) | 14.2 | 2 |
| Gly (with acid) | 2.9 | 2 |

## Claims

1. A pharmaceutical composition comprising glycopyrronium chloride at a dosage in the range of 0.5-100 µg per actuation dissolved in an HFA propellant and a co-solvent, **characterised in that** said composition contains an amount of 1M hydrochloric acid (HCl) in the range of 0.05-0.4 µg/µl.

2. A composition according to claim 1 wherein the range of 1M HCl is 0.19 - 0.25 µg/µl.

3. A composition according to claim 1 or claim 2 wherein the co-solvent is ethanol.

4. A composition according to any preceding claim wherein glycopyrronium chloride is at dosage in the range 1-40 µg per actuation.

5. A composition according to any preceding claim wherein glycopyrronium chloride is at a dosage in the range 5-26 µg per actuation.

6. A composition according to any preceding claim wherein glycopyrronium chloride is at a dosage of 25 µg per actuation.

7. A composition according to any preceding claim that additionally comprises one or more pharmaceutically active ingredients selected from the group consisting of beta-2-agonists, corticosteroids, antimuscarinic agents, and phosphodiesterase (IV) inhibitors.

8. A composition according to claim 7 that additionally comprises formoterol fumarate.

9. A composition according to claim 7 or claim 8 that additionally comprises beclometasone dipropionate.

10. A metered dose inhaler comprising a pharmaceutical composition as defined in any preceding claim.

11. A kit-of-parts comprising the pharmaceutical composition as defined in claim 1 and further comprising one or more pharmaceutically active ingredients for separate, sequential or simultaneous administration, wherein said pharmaceutically active ingredients are selected from the group consisting of beta-2-agonists, corticosteroids, antimuscarinic agents, and phosphodiesterase (IV) inhibitors.

12. Use of a pharmaceutical composition as defined in any of claims 1 to 9 in the manufacture of a medicament for use in the treatment or prophylaxis of a respiratory disease, such as asthma and COPD.

13. A pharmaceutical composition according to any of claims 1 to 9 for use in the prevention or treatment of asthma and COPD.

14. A method of filling an aerosol canister with the pharmaceutical composition as defined in any of claims 1 to 9, comprising the steps of:
a) preparing a solution comprising glycopyrronium chloride, a co-solvent, a mineral acid and optionally a low volatility component;
b) filling the open canister with the solution;
c) placing the valve onto the canister and crimping; and
d) pressure-filling the canister with HFA propellant through the valve.

## Patentansprüche

1. Pharmazeutische Zusammensetzung, umfassend Glycopyrroniumchlorid in einer Dosierung im Bereich von 0,5-100 µg pro Sprühstoß, aufgelöst in einem HFA-Treibmittel und einem Co-Lösungsmittel, **dadurch gekennzeichnet, dass** die Zusammensetzung 1 M Salzsäure (HCl) in einer Menge im Bereich von 0,05-0,4 µg/µl enthält.

2. Zusammensetzung nach Anspruch 1, wobei der Bereich an 1 M HCL 0,19-0,25 µg/µl beträgt.

3. Zusammensetzung nach Anspruch 1 oder Anspruch 2, wobei das Co-Lösungsmittel Ethanol ist.

4. Zusammensetzung nach einem der vorstehenden Ansprüche, wobei die Dosierung an Glycopyrroniumchlorid im Bereich von 1-40 µg pro Sprühstoß liegt.

5. Zusammensetzung nach einem der vorstehenden Ansprüche, wobei die Dosierung an Glycopyrroniumchlorid im Bereich von 5-26 µg pro Sprühstoß liegt.

6. Zusammensetzung nach einem der vorstehenden Ansprüche, wobei die Dosierung an Glycopyroniumchlorid 25 µg pro Sprühstoß beträgt.

7. Zusammensetzung nach einem der vorstehenden Ansprüche, zusätzlich umfassend einen oder mehrere pharmazeutisch wirksame Inhaltsstoffe, ausgewählt aus der Gruppe, bestehend aus beta-2-Agonisten, Corticosteroiden, Antimuskarinika und Phosphodiesterase-(IV)-Inhibitoren.

8. Zusammensetzung nach Anspruch 7, die zusätzlich Formoterolfumarat umfasst.

9. Zusammensetzung nach Anspruch 7 oder Anspruch 8, die zusätzlich Beclometasondipropionat umfasst.

10. Dosierinhalator, umfassend eine pharmazeutische Zusammensetzung, wie in einem der vorstehenden Ansprüche definiert.

11. Kit, umfassend die pharmazeutische Zusammensetzung, wie in Anspruch 1 definiert, und des Weiteren umfassend einen oder mehrere pharmazeutisch wirksame Inhaltsstoffe für die separate, sequentielle oder simultane Verabreichung, wobei die pharmazeutisch wirksamen Inhaltsstoffe ausgewählt sind aus der Gruppe, bestehend aus beta-2-Agonisten, Corticosteroiden, Antimuskarinika und Phosphodiesterase-(IV)-Inhibitoren.

12. Verwendung einer pharmazeutischen Zusammensetzung, wie in einem der Ansprüche 1 bis 9 definiert, zur Herstellung eines Medikaments zur Verwendung bei der Behandlung oder Vorbeugung einer Atemwegserkrankung, wie Asthma und COPD.

13. Pharmazeutische Zusammensetzung nach einem der Ansprüche 1 bis 9 zur Verwendung bei der Vorbeugung oder Behandlung von Asthma und COPD.

14. Verfahren zum Befüllen eines Aerosolbehälters mit der pharmazeutischen Zusammensetzung, wie in einem der Ansprüche 1 bis 9 definiert, umfassend die Schritte:
a) Herstellung einer Lösung, umfassend Glycopyrroniumchlorid, ein Co-Lösungsmittel, eine Mineralsäure und gegebenenfalls eine geringflüchtige Komponente,
b) Füllen des offenen Behälters mit der Lösung,
c) Platzieren des Ventils auf dem Behälter und Crimpen, und
d) Druckbefüllen des Behälters mit HFA-Treibmittel über das Ventil.

## Revendications

1. Composition pharmaceutique comprenant du chlorure de glycopyrronium à un dosage compris entre 0,5 et 100 µg par actionnement dissous dans un gaz propulseur HFA et un cosolvant, **caractérisée en ce que** ladite composition contient une quantité d'acide chlorhydrique (HCl) 1 M comprise entre 0,05 et 0,4 µg/µl.

2. Composition selon la revendication 1, dans laquelle la quantité de HCl 1 M est comprise entre 0,19 et 0,25 µg/µl.

3. Composition selon la revendication 1 ou 2, dans laquelle le cosolvant est l'éthanol.

4. Composition selon l'une quelconque des revendications précédentes, dans laquelle le chlorure de glycopyrronium est présent à un dosage compris entre 1 et 40 µg par actionnement.

5. Composition selon l'une quelconque des revendications précédentes, dans laquelle le chlorure de glycopyrronium est présent à un dosage compris entre 5 et 26 µg par actionnement.

6. Composition selon l'une quelconque des revendications précédentes, dans laquelle le chlorure de glycopyrronium est présent à un dosage de 25 µg par actionnement.

7. Composition selon l'une quelconque des revendications précédentes, qui comprend en plus un ou plusieurs ingrédients pharmaceutiques actifs choisis dans le groupe constitué par les agonistes bêta-2, les corticostéroïdes, les agents antimuscariniques et les inhibiteurs de la phosphodiestérase (IV).

8. Composition selon la revendication 7, qui comprend en plus du fumarate de formotérol.

9. Composition selon la revendication 7 ou 8, qui comprend en plus du dipropionate de béclométasone.

10. Inhalateur-doseur comprenant une composition pharmaceutique selon l'une quelconque des revendications précédentes.

11. Kit d'éléments comprenant la composition pharmaceutique selon la revendication 1 et comprenant en plus un ou plusieurs ingrédients pharmaceutiques actifs pour l'administration séparée, séquentielle ou simultanée, dans lequel lesdits ingrédients pharmaceutiques actifs sont choisis dans le groupe constitué par les agonistes bêta-2, les corticostéroïdes, les agents antimuscariniques et les inhibiteurs de la phosphodiestérase (IV).

12. Utilisation d'une composition pharmaceutique selon l'une quelconque des revendications 1 à 9, dans la fabrication d'un médicament pour l'utilisation dans le traitement ou la prophylaxie d'une maladie respiratoire telle que l'asthme et la broncho-pneumopathie chronique obstructive (BPCO).

13. Composition pharmaceutique selon l'une quelconque des revendications 1 à 9, pour l'utilisation dans la prévention ou le traitement de l'asthme et de la BPCO.

14. Procédé de remplissage d'une cartouche d'aérosol avec la composition pharmaceutique selon l'une quelconque des revendications 1 à 9, comprenant les étapes consistant à :
a) préparer une solution comprenant du chlorure de glycopyrronium, un cosolvant, un acide minéral et facultativement un composant faiblement volatil ;
b) remplir la cartouche ouverte avec la solution ;
c) placer la valve sur la cartouche et la sertir ; et
d) remplir sous pression la cartouche avec le gaz propulseur HFA par le biais de la valve.
